# EUROPEAN PATENT SPECIFICATION

(11) **EP 0 857 177 B1**
(45) Date of publication and mention of the grant of the patent: **23.04.2003**
(21) Application number: 96934590.9
(22) Date of filing: 09.10.1996
(51) Int. Cl.: C07K 16/00, C12N 15/86

(54) **PROCESS FOR THE PREPARATION OF A SINGLE CHAIN FV ANTIBODY FRAGMENT**
VERFAHREN ZUR HERSTELLUNG EINES EINKETTIGEN FV ANTIKÖRPERFRAGMENTS
PROCEDE DE PREPARATION D'UN FRAGMENT D'ANTICORPS FV A SIMPLE CHAINE

(30) Priority: 20.10.1995 EP 95810652
(43) Date of publication of application: 12.08.1998
(73) Proprietor: Novartis AG, 4056 Basel (CH); Novartis Pharma GmbH, 1230 Wien (AT)
(72) Inventor: KRETZSCHMAR, Titus, DK-3500 Varlose (DK); GEISER, Martin, CH-4106 Therwil (CH)
(74) Representative: Becker, Konrad
(86) International application number: EP9604376
(87) International publication number: WO97015601

(56) References cited:
- EP-A- 0 338 745
- EP-A- 0 505 207
- WO-A-91/16353
- WO-A-93/01296
- WO-A-93/08300
- WO-A-93/12246
- J.BIOL.CHEM., vol. 266, no. 25, 1991, pages 16343-49, XP002024003 LAROCHE ET AL: "Characterization of a recombinant single-chain molecule comprising the variable domains of a monoclonal antibody specific for human fibrin d-dimer"
- CRITICAL REVIEWS IN IMMUNOLOGY, 1992, vol. 12, no. 3-4, 1992, pages 125-168, XP000616488 WRIGHT A ET AL: "GENETICALLY ENGINEERED ANTIBODIES - PROGRESS AND PROSPECTS"
- EUROPEAN JOURNAL OF BIOCHEMISTRY, vol. 196, no. 3, 1991, pages 623-629, XP000616140 INGLEY E. ET AL.: "PRODUCTION AND PURIFICATION OF RECOMBINANT HUMAN INTERLEUKIN-5 FROM YEAST AND BACULOVIRUS EXPRESSION SYSTEMS" cited in the application
- "BACULOVIRUS EXPRESSION VECTORS" published 1994 by Oxford University Press, Ed.: O'Reilly et al., pages 35-37.

## Description

The present invention relates to a process for preparing Fv antibody fragments by recombinant DNA technology. In particular, the production of single chain Fvs (scFvs) in insect cells in culture using a Baculovirus expression system is described. The process of the invention yields a high percentage of correctly folded monomeric scFv which can easily be isolated.

### Background to the Invention

Engineering of antibody genes by molecular biological methods has led to a wealth of designer antibodies and thereof derived smaller antigen-binding fragments (for review: Winter, G. and Milstein, C. (1991), Nature 349, 293-299; Wright *et al*. (1992) Crit. Rev. Immunol. 12, 125-168). The Fv is the smallest antibody fragment which displays the antigen binding characteristics of whole immunoglobulins, consisting of the variable domains of heavy (V_{H}) and light (V_{L}) chains. Although difficult to produce by proteolytic cleavage of natural immunoglobulins, Fvs are particularly suitable for production by recombinant DNA approaches in view of their small size.

The heavy and light chains in Fvs are normally held together by non-covalent forces, which predisposes Fvs to dissociation at low concentrations and, most importantly, under physiological conditions. In order to address this problem, and moreover further increase the suitability of Fv molecules for recombinant DNA production, so-called single chain Fvs have been developed (Bird *et al*. (1988) Science, **242,** 423-426; Huston *et al*. (1988) PNAS (USA), **85,** 5879-5883). scFvs consist of a single polypeptide chain in which the V_{H} and V_{L} domains of a normal Fv molecule are held together covalently by an integral polypeptide linker. In general, the linkers are selected on their ability to link the antibody chains together without compromising binding ability, meaning that they must be of a length appropriate to span the gap between the C-terminus of one V domain and the N-terminus of the other (estimated at 35 Å), which is around 15 amino acids. Typically, multiples of the Gly-Gly-Gly-Gly-Ser are employed (such as (Gly₄ Ser)₃; Huston *et al*., 1988).

Binding affinity studies conducted with scFvs have traditionally demonstrated that binding is equivalent to that of the "parent" antibody, while several advantages associated with Fvs, mostly deriving from their small size, have indicated scFvs particularly for *in vivo* therapeutic and diagnostic applications. For example, Fvs show rapid clearance from plasma and extravascular space, but efficient accumulation in targeted tissues such as tumours. At the same time, Fvs do not accumulate in kidney, in contrast to Fabs, which do. Furthermore, Fvs demonstrate superior tumour penetration to whole immunoglobulins, distributing uniformly throughout tumour tissue. For a review of the properties of Fvs and scFvs see Raag and Whitlow (1995) FASEB J., **9**, 73-80 and references cited therein.

One problem which has been identified with scFvs is their tendency to associate into dimers and high molecular weight oligomers. Although this does not appear to reduce binding performance (in fact, it improves it), it does have a bearing on the many clinical advantages associated with the small size of Fvs. In order to maximise tissue penetration and clearance properties, it is essential to avoid the formation of high molecular weight oligomers. Dimers are likewise undesirable.

Oligomers of scFvs are believed to form when V_{H} and V_{L} domains from different scFv molecules associate with each other, intermolecularly, rather than intramolecularly within the scFv molecule. Thus, the oligomers are true oligomers with unaltered binding specificity (so long as the Fv population is not heterogeneous) rather than mere aggregates and for this reason they display increased avidity in binding to many antigens. This is often results in over-estimated apparent binding affinity, especially in ELISA assays, due to a mixture of affinity and avidity effects.

Various systems suitable for the production of scFvs are available to one of skill in the art. Initially, the interest in these small antibody fragments was stimulated when heterologous expression in *E.coli* could be demonstrated (Bird *et al*., 1988; Huston *et al*., 1988; Skerra and Plückthun (1988) Science **240,** 1038-1041). Besides in *E.coli*, scFv have also been expressed and purified from *Bacillus subtilis*, mammalian cells, and insect cells (Laroche *et al*., (1991) J. Biol. Chem. **266,** 16343-16349).

Systems for production of antibody molecules, including scFvs, in insect cells have been reviewed by Potter *et al*., (1993) Intern. Rev. Immunol. **10,** 103-112. The most widespread expression system used in insect cells is based on baculovirus vectors. A baculovirus expression vector is constructed by replacing the polyhedrin gene of baculovirus, which encodes a major structural protein of the baculovirus, with a heterologous gene, under the control of the strong native polyhedrin promoter. Cultured insect host cells are infected with the recombinant virus, and the protein produced thereby can be recovered from the cells themselves or from the culture medium if suitable secretion signals are employed.

Laroche *et al*., in the experiments reported in their 1991 publication, were able to purify 4.8mg of scFv per litre of culture medium by ion exchange chromatography and gel filtration. This contrasts with the 100-150mg/litre available through other secretion systems employing affinity purification or Ni²⁺ chelate chromatography in order to isolate the scFvs (Pack *et al*., (1995) J. Mol. Biol. **246,** 28-34; Burks and Iverson, (1995) Biotechnol. Prog. **11,** 112-114; Ridder *et al*., (1995) Bio/Technology 13, 255-260). Laroche *et al*. did not comment on the issue of scFv oligomerisation.

We have now shown that insect cell expression systems analogous to that used by Laroche *et al*. produce a very high percentage of monomeric Fv which is easily purified from culture media without the use of affinity purification or Ni²⁺ chelate chromatography, which are expensive and difficult to operate industrially. Moreover, we have been successful in obtaining 32mg/litre of scFv from insect cell culture.

### Summary of the Invention

According to the invention, there is provided a process for producing single chain Fv antibody molecules in insect host cells according to claim 1.

### Detailed Description of the Invention

For the purposes of the present invention, "single chain Fv antibody molecule", also referred to as scFv, is to be understood to comprise V_{H} and V_{L} domains of an antibody molecule covalently linked together by a polypeptide linker or spacer to form a single polypeptide chain which is capable of folding to form a functional antigen-binding molecule. Without prejudice to the foregoing, the term is to be interpreted in accordance with its signification in the art. An example of a single chain antibody is found in reference WO 91/16353. It is an advantage of the invention that insect cell expression systems secrete a high percentage of monomeric Fv. The low percentages of dimeric and multimeric Fvs can be removed by size separation. Numerous methods are available in the art for separating polypeptides on the basis of size, such as chromatography and gel electrophoresis. Preferred are methods which perform a purification function as well as a size separating function, whilst not introducing unacceptable contaminants. Thus, methods such as step or continuous gradient centrifugation, particularly using sucrose gradients, dialysis techniques using controlled-pore membranes and membrane (Amicon) centrifugation are preferred. Especially preferred, however, is size exclusion chromatography, typically performed using porous beads as the chromatographic support. Size exclusion chromatography is, for example, described by Stellwagen, in Deutscher (1990) Guide to Protein Purification, Academic Press, Inc., San Diego, CA, USA, 317-328.

Advantageously, the monomeric scFv antibody molecules are purified before being size separated from the multimeric forms. By "purified", it is meant that the scFv antibody molecules are separated from other products, proteinaceous or otherwise, present in the crude preparation of secreted antibody molecules. Such further separation techniques may be required depending on the performance of the size separation step in purifying the scFv from unwanted materials, the nature of the medium used and the intended use of the final monomeric scFv product Protein purification techniques useful in the present invention are entirely conventional. Protein purification is, for example, described in general in Deutscher (1990).

Suitable purification methods include chromatography in general, based on separation by charge difference and/or size difference, such as ion exchange chromatography using an exchange group such as DEAE or CM bound to a solid phase packing material such as cellulose, dextran, agarose or polystyrene. Other methods include hydroxyapatite column chromatography (see, for example, Gorbunoff, (1985) Methods in Enzymology, **117,** 370-380), and general affinity chromatography using glass beads or reactive dyes as affinity agents.

Advantageously, cation exchange chromatography may be employed, such that protein elution can be tailored to take into account the known or estimated pI of the scFv in question. The pI for any scFv may be determined experimentally, by isoelectric focusing. In this manner, it is possible selectively to elute from the cation exchange resin those proteins having a pi at or around that of the scFv, which results in a high degree of purification.

Insect cells suitable for use in the method of the invention include, in principle, any lepidopteran cell which is capable of being transformed with an expression vector and expressing heterologous proteins encoded thereby. In particular, use of the Sf cell lines, such as the *Spodoptera frugiperda* cell line IPBL-SF-21 AE (Vaughn *et al*., (1977) In Vitro, **13,** 213-217) is preferred. The derivative cell line Sf9 is particularly preferred. However, other cell lines, such as *Tricoplusia ni* 368 (Kurstack and Marmorosch, (1976) Invertebrate Tissue Culture Applications in Medicine, Biology and Agriculture. Academic Press, New York, USA) may be employed. These cell lines, as well as other insect cell lines suitable for use in the invention, are commercially available (e.g. from Stratagene, La Jolla, CA, USA).

A recombinant baculovirus can also be used in insect control such as described in EP 505207.

Expression vectors suitable for use in the method of the invention include all vectors which are capable of expressing foreign proteins in insect cell lines. In general, vectors which are useful in mammalian and other eukaryotic cells are also applicable to insect cell culture. Baculovirus vectors, specifically intended for insect cell culture, are especially preferred and are widely obtainable commercially (e.g. from Invitrogen and Clontech). Other virus vectors capable of infecting insect cells are known, such as Sindbis virus (Hahn *et al*., (1992) PNAS (USA) **89,** 2679-2683). The baculovirus vector of choice (reviewed by Miller (1988) Ann. Rev. Microbiol. **42,** 177-199) is *Autographa californica* multiple nuclear polyhedrosis virus, AcMNPV.

Typically, the heterologous gene replaces at least in part the polyhedrin gene of AcMNPV, since polyhedrin is not required for virus production. In order to insert the heterologous gene, a transfer vector is advantageously used. Transfer vectors are prepared in *E. coli* hosts and the DNA insert is then transferred to AcMNPV by a process of homologous recombination.

Molecular biology techniques useful for practising the present invention are entirely conventional and well known to those of ordinary skill in the art. They are reviewed generally in Sambrook, (1989) Molecular Cloning; A Laboratory Manual, Cold Spring Harbor, NY, USA. Baculovirus techniques are equally standard and well known in the art, and are reviewed in O'Reilly *et al*., (1994) Baculovirus expression vectors; A laboratory manual, Oxford University Press Inc., NY, USA, as well as in literature published by suppliers of commercial baculovirus kits (e.g. Pharmingen).

The expression vector used in the present invention comprises the necessary regulatory sequences useful for expression and secretion of the encoded heterologous polypeptide. The entire polyhedrin untranslated leader sequence may be used, upstream of the heterologous gene. However, alternative signal peptides effective in insect cells may be employed. The use of the baculovirus envelope glycoprotein GP67 (also known as GP64) is preferred. The heterologous gene is preferably under the control of the strong polyhedrin promoter, although the use of alternative promoters and enhancers effective in insect cells is envisaged. For example, the use of promoters, enhancers and/or signal sequences derived from insect viruses other than baculoviruses, or from insect genes, is possible. The promoter and/or enhancer may be a regulatable promoter and/or enhancer, permitting controlled induction of gene expression.

Culture media suitable for insect cell culture are known in the art and typically contain 5-10% fetal calf serum (FCS). However, the present invention employs culture media which are serum-free, such media are known (Ingley *et al*., (1991) Eur. J. Biochem. **196,** 623-629; DiSorbo *et al*., (1991) Focus **13,** 16-18). Through the use of serum - free media the contamination of the antibody product with material derived from the FCS, such as bovine immunoglobulin, is avoided. The use of entirely protein-free medium is also possible.

Due to the use of signal sequences in the heterologous gene construct, the scFv molecule will be secreted into the culture medium. Isolation and purification from . culture medium is considerably easier than purification from cell lysates, as cellular material does not need to be removed from the preparation. The avoidance of affinity purification is thereby permitted. The preferred isolation method for use in connection with the invention comprises dialysis and cation exchange chromatography followed by size exclusion chromatography. The size exclusion step is required in order to separate the monomeric scFv, produced in large percentage by the method of the present invention, from dimeric or oligomeric contaminants.

The invention is further described, for the purposes of illustration only, in the following examples, of which example 2 does not fall within the scope of the claims.

### Example 1: Construction of a baculovirus vector encoding scFv

We have chosen an insect cell-based expression system to examine its efficacy and convenience for the production and purification of scFv. The DNA coding for the fusion αphOx15-scFv::c-myc tag::amber codon is isolated by standard PCR with the Vent DNA polymerase (Biolabs) from the template pHEN1::αphOx15-scFv (Hoogenboom, *et al*., (1991) Nucl. Acids Res. **19,** 4133-4137; Marks, *et al*., (1991). J. Mol. Biol. **222,** 581-597) using the following primers: 5' ATA TCC CGG GCA GGT GCA GCT GGT GCA GTC and 5' ATA TGA ATT CTC TAT GCG GCC CCA TTC AGA (restriction sites for Sma I and EcoR I are underlined). The gel purified fragment is cloned into the transfer vector pAcGP67A (Pharmingen). The correct insertion of the scFv gene is confirmed by sequencing.

The E.coli strain HB101 (F⁻ hsdS20 [r_{b}⁻m_{b}⁺] supE44 ara14 galK2 lacY1 proA rpsL20 (Str^{R}) xyl-5 mtl-1 recA13 mcrB thi-1; Invitrogen) is employed for all handling and manipulation steps of the vector DNA prior to its transfection into insect cells. A recombinant transfer vector DNA is prepared with the Plasmid Maxi Kit (Qiagen) and sequenced with the T7 Sequencing Kit with Deaza G/A 17 Sequencing Mixes (Pharmacia). Transfection of Sf9 cells is performed according to the BaculoGold Transfection Kit manual (Pharmingen). The Sf9 cells are cultured in TC100 medium supplemented with 10% of neat-inactivated FCS, gentamycin (50 µg/ml), and amphotericin B (2.5 µg/ml, all material purchased from Gibco). After co-transfection of Sf9 cells with the transfer vector pAcGP67::anti-phOx-scFv and linearized baculovirus DNA, and after one single round of plaque purification the recombinant virus is amplified and titrated using the end-point dilution method according to standard protocols (O'Reilly *et al*., (1994). Five randomly picked virus plaques are analysed for scFv gene expression. The viruses are checked for their ability to express the scFv gene in small scale expression trials which are judged by immunoblotting. All five virus isolates lead to comparable amounts of anti-phOx-scFv released into the culture medium as judged by immunoblotting with an anti-c-myc antibody. One arbitrarily chosen virus is amplified to large scale to yield a stock of 3.3 x 10⁷ plaque forming units/ml which is applied for all further expression experiments. The amplified virus stocks are stored at 4°C in the dark.

### Example 2: Insect cell culture in a bioreactor

In pre-experiments it is noticed that a MOI of one is sufficient for obtaining maximum levels of scFv secretion. Sf9 cells are maintained in ExCell-400 medium (JRH Biosciences) supplemented with 5% FCS at 27°C in a spinner flask rotating at 70 rpm. Cells are counted using a hemocytometer and viability assessed by trypan blue exclusion (0.05% [w/v]). Cell culture is scaled-up in a 10 liter anchor-stirred glass bioreactor with a water jacket. The bioreactor is inoculated with 2.7 x 105 cells/ml in 3 litres of ExCell-400 medium (pH 6.3) supplemented with 5% FCS and 50 µg/ml gentamycin. Oxygen (45%) and temperature (27°C) are continuously monitored. The cells are grown in the bioreactor for 3 days to a density of 2 x 10⁶ cells/ml and then infected with the recombinant virus preparation at a MOI of one. At the same time the volume is increased to 6 litres with TC-100 medium supplemented with 5% FCS and 50 µg/ml gentamycin. The kinetics of expression are measured by harvesting aliquots of the cell supernatant at required time intervals post-infection. The supernatant is harvested 3 days post-infection by centrifugation for 20 min at 200 x g. After separation by SDS-PAGE under reducing conditions (2.5% [v/v] 2-mercaptoethanol) anti-c-myc antibody reactive protein bands are visualised by immunoblotting. Expression of secreted scFv reaches saturation at about 72 h. No degradation products of the scFv can be detected.

For scaling up the scFv production a 6 litres culture of infected insect cells is run. The expression kinetics in the bioreactor and in cell culturing of adherent insect cells in flasks are nearly identical. At the time point of harvest (72h) 93% of the cells are still viable as determined by trypan blue exclusion. The supernatant is supplemented with 0.05% [w/v] NaN₃ and stored at -20°C for further purification.

### Example 3: Cation Exchange Chromatography

From our own set of data of genomic V_{H} (n=16) and V_{L} (n=12) sequences and prediction of their isoelectric points using the Sequence Analysis Software Package from the Genetics Computer Group (1991), it is possible to determine the basic pi of these fragments. For assembled scFv we calculate an average pI of 8.4 ± 1.5. The pI of the anti-phOx-scFv is 9.8, and by isoelectric focusing it is possible to confirm experimentally that its pI is more alkaline than 9.3. Hence, cation exchange chromatography at pH 6.0 is employed to separate the bulk of BSA originating from the cell culture medium from the scFv. The anti-phOx-scFv always co-migrates with the BSA. Only disruption of non-specific interactions between the scFv and BSA by addition of Tween-20 to the buffers allow restoration the expected running behaviour.

An aliquot of the supernatant of the insect cell culture is dialysed in a membrane tube (Spectra/Por 2 with a molecular weight cut-off of 12-14 kD, Spectrum) against 50 mM malonic acid pH 6.0. After supplementing with 0.05% [w/v] NaN₃ and 0.22 µm-filtration (Millex-GV, Millipore), 0.1% [v/v] Tween-20 is added. 25 ml of this sample are applied to a 3 ml-SP Sepharose Fast Flow (Pharmacia) glass column (Bio-Rad, inner diameter of 1 cm). The run is driven by the ConSep LC100 controlling device (Millipore) connected to the pump P-1 (Pharmacia) at a speed of 0.9 ml/min at room temperature. Proteins are eluted with a linear salt gradient (25 ml) from 0 to 0.7 M NaCl. The gradient is checked by titration of the Cl⁻-ions with AgNO₃. Fraction volumes of 0.7 ml are collected. All fractions are examined for the presence of the scFv both by ELISA exploiting the c-myc flag sequence and by SDS-PAGE (Laemmli, U.K. (1970) Nature **227,** 680-685) developed by silver staining according to Blum et al., (1987) Electrophoresis **8,** 93-99. By chemiluminescence ELISA and by SDS-PAGE with silver staining one peak of anti-phOx-scFv is detected in fractions eluted between 130-240 mM NaCl. The vast excess of BSA is found in the flow through. 85% of the anti-phOx-scFv activity is recovered, as judged by ELISA.

### Example 4: Size exclusion chromatography

SDS-PAGE analysis of the anti-phOx-scFv fraction after cation exchange shows that all contaminating proteins with a molecular weight up to 50 kDa have been removed. Thus, size exclusion chromatography is chosen as next purification step. A Sephadex G100 superfine gel is used for separation albeit a Sephadex G75 superfine gel works almost equally well.

A Sephadex G100 superfine (Pharmacia) column (inner radius of 7.5 mm, gel bed height of 80 cm) equilibrated in PBS / 0.05% [v/v] Tween-20 / 0.05% [w/v] NaN₃ is used for size exclusion. The run is performed at 4°C at gravity flow. The height difference between the reservoir and the outlet is 205 cm. 1 ml of the pooled sample obtained after cation exchange is applied to the column. The column is developed at a speed of 1.4 ml/h. The collected fraction size is about 1.1 ml. All fractions are checked for ELISA signals and for presence of a protein band around 30 kD. In order to detect impurities in the final product an aliquot of the pool of fractions containing the anti-phOx-scFv monomers is concentrated using a centricon-10 concentrator device (10 kDa molecular weight cut-off, Amicon).

The peak of the ELISA signal and the appearance of a clearly visible 32 kDa band of the anti-phOx-scFv after SDS-PAGE and after immunoblotting are shifted against each other by 16 fractions corresponding to 18.5 ml of the effluent. The ELISA signal emerges at a point in the chromatogram where the 60-90 kDa proteins are found. SDS-PAGE with subsequent silver staining as well as immunoblotting of this 60-90 kDa fraction reveals a faint band at 32 kDa. With a closer look at the ELISA data a second very weak peak can be detected which exactly matches those fractions in which the main part of the anti-phOx-scFv is located, as seen in SDS-PAGE. From these results it can be deduced that the dominant ELISA peak is due to a multivalent interaction of coated phOx_{20.5}BSA with anti-phOx-scFv dimers while scFv monomers are eluted 16 fractions later.

### Example 5: Culture in Serum-Free Media

Sf-9 cells are used to deomstrate the expression of single-chain Fv in accordance with the invention is serum-free media. Cells are seeded at 3.5x10⁵ cells/ml in ExCell-400 serum-free medium (JRH Biosciences; Sf-900 II from Gibco can also be used). When the cells reach a desity of 2x10⁶ cells/ml, they are infected with recombinant baculovirus at an MOI of 1. The following nutrients are added: glucose (2g/l), glutamine (2mM), Gibco 10x lipid concentrate (10ml/l) and Gibco 50x yeastolate (20ml/l). The cells are cultured as above and the expressed Fv protein similarly recovered.

The concentration of secreted Fv protein in the culture medium is found to be increased 5 tor 6 fold with respect to culture in serum-containing medium.

### Example 6: Analysis of scFv

### Immunoblotting

After separation by 0.1% SDS-12% PAGE the proteins are transferred to an Immobilon-P membrane (Millipore, 0.45 µm) under standard conditions (Ausubel et al., (1994) Current protocols in molecular biology. John Wiley & Sons Inc., USA). The blocked membrane is first reacted with protein G-purified mouse anti-c-myc antibody 1-9E10 (Evan et al., (1985) Mol. Cell. Biol. 5, 3610-3616) at a concentration of 3 µg/ml in TBS / 0.05% [v/v] Tween-20 / 5% [w/v] non-fat dry milk (buffer A). Thereafter, a goat anti-mouse immunoglobulin G antibody conjugated to horseradish peroxidase (Bio-Rad) is added at a 1:1000 dilution in buffer A. The colour development is started by incubation with a Co²⁺-supplemented diaminobenzidine solution (Harlow, E. and Lane, D. (1988) Antibodies. A laboratory manual. Cold Spring Harbor Laboratory, Cold Spring Harbor, NY, USA).

Aliquots of each step of purification are applied to SDS-PAGE. Analysis of the gels shows that the preponderating BSA contamination is successfully removed by cation exchange chromatography at pH 6.0. By size exclusion chromatography the high molecular weight impurities are efficiently separated from the anti-phOx-scFv. A concentrated aliquot of the ELISA-active fraction of the anti-phOx-scFv after size exclusion chromatography is tested, showing that this fraction comprised 60-90 kDa proteins. A band at 32 kDa representing disassembled anti-phOx-scFv dimers is barely detectable demonstrating that only minute amounts of scFv dimers had been generated. The high purity (>98%) of the obtained scFv monomer preparation is demonstrated after additional concentration by ultrafiltration.

Amino-terminal protein sequencing on a Hewlett Packard G1005A protein sequencer confirms the homogeneity of the scFv monomer product. The GP67 signal sequence is completely removed and no further N-terminal degradation has taken place.

The final scFv concentration is determined by two methods: i) the bicinchoninic acid protein assay with BSA as standard (Pierce) which yields a concentration of 15-21 µg/ml, and ii) the absorption at 280 nm which indicates an anti-phOx-scFv concentration of 22 µg/ml based upon a calculated extinction coefficient of 59000 l /(mol x cm). Thus, the overall recovery of purified anti-phOx-scFv monomers is 32 mg per litter of supernatant from insect cell culture in the bioreactor (10⁹ cells/l). The storage of scFv monomers at 4°C at concentrations between 20-40 µg/ml does not lead to multimerization.

### Chemiluminescence ELISA

The wells of a Microlite 2-immunoassay plate (Dynatech Laboratories) are coated at pH 8.1 with either 3.8 µg of phOx_{20.5}BSA prepared as described by Mäkelä *et al*., (1978) J. Exp. Med. **148,** 1644-1660, or 3.8 µg of BSA as negative control. After blocking with buffer A the samples are applied for 2h at 37°C. The wells are washed with ice-cold TBS / 0.05% [v/v] Tween-20, and the protein G-purified mouse anti-c-myc antibody 1-9E10 is added at a concentration of 3 µg/ml, followed by a goat anti-mouse immunoglobulin G antibody conjugated to horseradish peroxidase (see above). The peroxidase activity is quantified with the chemiluminescence substrate luminol (BM Chemiluminescence ELISA Reagent, Boehringer Mannheim). Readings of emitted light are recorded as RLU using a microtiter plate luminometer (ML3000, Dynatech Laboratories).

In order to assess the binding properties of the anti-phOx-scFv, we determined by competition ELISA the IC₅₀ values for mono- and multivalent binding. In both cases 3,8 µg of phOx_{20.5}BSA are coated per well of a microtiter plate. For measuring the multivalent interactions, the crude preparation of scFv dimers after size exclusion chromatography is equilibrated with increasing concentrations of phOx_{20.5}BSA prior to addition to the wells. For measuring the monovalent interaction, we used phOx₁ BSA for competition. The competition assays results in IC₅₀ which differed by a factor of 353: IC₅₀ (phOx_{20.5}BSA) = 3.4 x 10⁻⁷ M and IC₅₀ (phOx₁BSA) = 1.2 x 10⁻⁴ M. The phOx₁BSA conjugate is prepared by limiting the input of 4-ethoxymethylene-phOx to the coupling reaction (Mäkelä et al. (1978); Kretzschmar, *et al*., (1995) Anal. Biochem. **224,** 413-419).

Neither the reaction of monomeric anti-phOx-scFv with coated phOx_{20.5}BSA nor the interaction of both monomeric and dimeric scFv with coated phOx₁BSA yield ELISA signals allowing an analysis. This indicates that the antigen phOx has to be offered in a high local density for efficient scFv binding.

### Surface plasmon resonance measurements

A second independent assay is employed to confirm the low affinity of the monovalent interaction between anti-phOx-scFv and phOx. Surface plasmon resonance signals (Kretschmann, E. and Raether, H. (1968) Z. Naturforsch. 23a, 2135-2136; Liedberg, *et al*., (1983) Sensors and Actuators **4,** 299-304) are recorded with a BlAcore machine (Pharmacia Biosensor AB; Jönsson, *et al*., (1991) BioTechniques **11,** 620-627; Chaiken, *et al*., (1992) Anal. Biochem. **201,** 197-210). Purified monomeric anti-phOx-scFv at a concentration of 40 µg/ml in 5 mM maionic acid pH 6.0 / 0.05% [v/v] Tween-20 is coupled to a CM5 sensor chip using the Amine Coupling Kit (Pharmacia) as described earlier (Johnsson, *et al*., (1991) Anal. Biochem. **198,** 268-277). Via amino groups, 2160 RU of the purified monomeric anti-phOx-scFv is immobilised to the sensor chip (i.e., about 2 ng/mm² chip surface; Stenberg, *et al*., (1991) J. Colloid Interface Sci. **143,** 513-526).The activation time is adjusted to 7 min at a flow rate of 5 µl/min. For immobilisation 35 µl of the scFv sample are injected to the chip at the same flow rate. All dilutions of the analytes phOx_{20.5}BSA and of the BSA control are prepared with one batch of the basic flow buffer HBS (10 mM HEPES, 150 mM NaCI, 3.4 mM EDTA, 0.05% [v/v] surfactant P-20 [Pharmacia], pH 7.4, degassed and 0.22 µm-filtered). Different concentrations of the analytes are injected at 2 µl/min for 15 min before applying HBS to initiate the dissociation. Data point sampling occurs every 2 seconds in the association and dissociation phases and in 10 to 60 s intervals in the equilibrium phase. The chip is regenerated by injection of 10 mM HCI for 3 min without loss of function. The data analysis is performed according to the methods and equations indicated in the BlAcore manual and supported by the Bioevaluation software (Pharmacia).

Since a notable background binding of BSA to the modified chip is observed, it is indispensable to record the scFv binding events for both analytes phOx_{20.5}BSA and BSA. By subtracting the RU obtained with BSA from those elicited with phOx_{20.5}BSA it is possible to calculate RU changes specifically induced by phOx. K_{D} can be derived from the differential RU measured at different phOx concentrations before dissociation is initiated. The inverted absolute value of the slope of the curve is transposed into molar units resulting in a K_{D} of 8.47 x 10⁻⁵ M (with an error of 12.6% for the slope).

## Claims

1. A process for producing single chain Fv antibody molecules in insect host cells comprising the steps of:
(i) transforming insect host cells with an expression vector comprising a DNA sequence encoding a single chain Fv antibody polypeptide and a signal peptide under the control of which the single chain Fv antibody molecule is secreted,
(ii) culturing said transformed insect host cells in vitro in serum-free medium,
(iii) subjecting the secreted single chain Fv antibody to a purification including at least one size separation step and
(iv) selecting the monomeric protein fraction wherein the vield of monomeric protein is greater than 32 mg per liter of culture medium.

2. A process according to claim 1 wherein the expression vector is a baculovirus expression vector.

3. A process according to claims 1 or 2 wherein the signal peptide Is a baculovirus envelope glycoprotein GP67 signal peptide.

4. Process according to any preceding claim wherein the secreted scFv antibody molecules are purified prior to the size separation step.

5. A process according to claims 1-3 wherein the host cells are of cells or a derivative thereof.

## Patentansprüche

1. Verfahren zur Herstellung von einkettigen Fv Antikörpermolekülen in Insektenwirtszellen, **gekennzeichnet durch** die folgenden Schritte
(i) Transformation von Insektewirtszellen mit einem Expressionsvektor, der eine DNA Sequenz enthält, welche für ein einkettiges Fv Antikörperpolypeptid und ein Signalpeptid kodiert, unter dessen Kontrolle das einkettige Fv Antikörpermolekül sekretiert wird.
(ii) Kultivierung der transformierten Insektenwirtszellen in vitro in serumfreiem Medium,
(iii) Unterziehung des sekretierten einkettigen Fv Antikörpers einer Reinigung, die zumindest einen Größentrennschritt umfaßt,
(iv) Auswahl der monomeren Proteinfraktion, worin die Ausbeute des monomeren Proteins größer als 32 mg pro Liter Kulturmedium ist.

2. Verfahren nach Anspruch 1. worin der Expressionsvektor ein Bakulovirusexpressionsvektor ist.

3. Verfahren nach einem der Ansprüche 1 oder 2, worin das Signalpeptid ein Signalpeptid des Hüllglycoproteins GP67 vom Baculovirus ist.

4. Verfahren nach einem der vorangehenden Ansprüche, worin die sekretierten scFv Antikörpermoleküle vor dem Größentrennschritt gereinigt werden.

5. Verfahren nach einem der Ansprüche 1-3, worin die Wirtszellen Sf Zellen oder ein Derivat hiervon sind.

## Revendications

1. Procédé de préparation de molécules d'anticorps Fv à une seule chaîne dans des cellules hôtes d'insecte, comprenant les étapes consistant à :
(i) transformer les cellules hôtes d'insecte avec un vecteur d'expression comprenant une séquence d'ADN codant pour un polypeptide d'anticorps Fv à une seule chaîne et un peptide signal sous le contrôle duquel la molécule d'anticorps Fv à une seule chaîne est sécrétée,
(ii) cultiver *in vitro* lesdites cellules hôtes d'insecte transformées dans un milieu sans sérum,
(iii) soumettre l'anticorps Fv à une seule chaîne sécrété à une purification comprenant au moins une étape de calibrage, et
(iv) sélectionner la fraction protéique monomère dans laquelle le rendement en protéine monomère est supérieur à 32 mg par litre de milieu de culture.

2. Procédé suivant la revendication 1, dans lequel le vecteur d'expression est un vecteur d'expression de baculovirus.

3. Procédé suivant la revendication 1 ou 2, dans lequel le peptide signal est le peptide signal GP67 de glycoprotéine d'enveloppe de baculovirus.

4. Procédé suivant l'une quelconque des revendications précédentes, dans lequel les molécules d'anticorps scFv sécrétées sont purifiées avant l'étape de calibrage.

5. Procédé suivant les revendications 1-3, dans lequel les cellules hôtes sont des cellules sf ou un dérivé de celles-ci.
